# EUROPEAN PATENT APPLICATION

(11) **EP 4 685 128 A1**
(43) Date of publication of application: **28.01.2026**
(21) Application number: 24774284.4
(22) Date of filing: 28.04.2024
(51) Int. Cl.: C07C 65/01, C07C 51/41, C07C 211/38, C07C 209/00, A61P 9/00, A61P 9/10, A61K 31/205

(54) **NOVEL 2-(1-HYDROXYPENTYL) BENZOIC ACID CYCLOALKYL AMINE SALT**

(30) Priority: 22.03.2023 CN 202310285308
(71) Applicant: Chengdu Shibeikang Biomedical Technology Co., Ltd., Chengdu, Sichuan 611731 (CN)
(72) Inventor: WANG, Xiaoyu, Chengdu, Sichuan 611731 (CN); ZHANG, Li, Chengdu, Sichuan 611731 (CN); FU, Haixia, Chengdu, Sichuan 611731 (CN); MOU, Xia, Chengdu, Sichuan 611731 (CN); JIANG, Jie, Chengdu, Sichuan 611731 (CN); LI, Lianming, Chengdu, Sichuan 611731 (CN)
(74) Representative: IP TRUST SERVICES
(86) International application number: PCT/CN2024/090342
(87) International publication number: WO 2024/193721

(57) **Abstract**

A cycloalkylammonium 2-(1-hydroxypentyl)benzoate salt of the structure represented by formula I, a preparation thereof, a pharmaceutical composition containing the compound as an active ingredient, and a use of the compound in the treatment and/or prevention of ischemic cardiovascular and cerebrovascular disorders, especially mild, moderate and severe acute ischemic strokes.

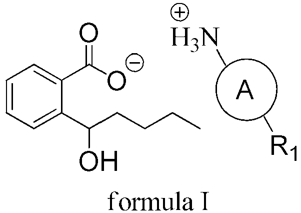

## Description

### TECHNICAL FIELD

The present invention belongs to the field of pharmaceutical technology and specifically relates to a novel 2-(1-hydroxypentyl) benzoate salt, a preparation process thereof, a pharmaceutical composition containing the compound as an active ingredient, and a use thereof in the treatment and/or prevention of ischemic cardiovascular and cerebrovascular disorders, especially mild, moderate and severe types of acute ischemic strokes.

### BACKGROUND ART

Acute ischemic stroke, cerebral thrombosis, cerebral embolism, coronary heart disease, angina pectoris, myocardial infarction and the like are common ischemic cardiovascular and cerebrovascular diseases. These diseases are conditions characterized by ischemic injury caused by thrombus formation triggered by various factors, leading to ischemic injury. In particular, ischemic cerebrovascular diseases, due to severe cerebrovascular stenosis, cerebral infarction, and/or cerebral thrombosis, result in vascular occlusion and insufficient blood supply to the brain, leading to neuronal damage and necrosis. Such diseases bring great pain and even pose life-threatening danger to patients. Consequently, drug research in this field has long been regarded as a frontier and a hotspot of scientists' attention.

Butylphthalide is a Class I new drug developed domestically in recent years for the treatment of ischemic cerebrovascular diseases. The currently available formulations are butylphthalide soft capsules and butylphthalide sodium chloride injections. The main mechanism of action is to improve the microcirculation of cerebral ischemic area, promote angiogenesis in the ischemic region, and increase cerebral blood flow in the ischemic area. Butylphthalide is a lipid-soluble compound that exhibits an oily liquid form at room temperature, and have a poor solubility in water, and a low solubility *in vivo.* These unfavorable physicochemical properties inevitably result in difficulties in its preparation process and low *in vivo* utilization, which in turn leads to insufficient drug efficacy. Therefore, the overall efficacy of butylphthalide in clinical applications is not high, and butylphthalide often needs to be used in combination with other drugs to enhance its effectiveness, which limits its scope of application in the clinical treatment of ischemic diseases.

Patent, published as CN1382682A first disclosed the preparation of 2-(α-hydroxy-pentyl)benzoate salt using DL-3-n-butylbenzyl as a lead compound and the use thereof, involving the salts of monovalent metal ions, divalent metal ions and organic bases, and specifically disclosed the salts of potassium, sodium, calcium, magnesium, zinc, benzylamine, benzylamine, morpholine, diethylamine. The specification further discloses in the pharmacology section the effects of the potassium salt on cerebral infarct area in rats with focal cerebral ischemia, its effects on platelet aggregation in rats, and its protective effects against ischemia-reperfusion-induced arrhythmias in isolated rat hearts, demonstrating that the potassium salt exerted beneficial effects in above experiments. Patent, published as CN1523003A provides a comprehensive comparision of various metal salts and organic amine/inorganic ammonium salts of 2-(α-hydroxypentyl)benzoic acid, among which potassium salt is highly hygroscopic, and exhibits greater toxicity, tert-butylamine salt and benzylamine salt are highly toxic; N'N-dibenzylethylenediamine salt exhibits lower toxicity, but its pharmacological efficacy is only comparable to that of the potassium salt. Additionally, it has a large molecular weight, which, on one hand, reduces absorption and, on the other hand, results in a larger single-dose specification, thereby reducing patient compliance. Therefore, currently available and in-progress projects on the market lack butylphthalide ring-opening derivative drugs with high safety, significant efficacy, and suitability for drug formulation.

Therefore, the further development of more effective drugs with stable solid forms, suitable for intravenous injection, for the treatment of ischemic cardiovascular and cerebrovascular diseases, particularly those targeting ischemic cerebrovascular diseases, represents a critical challenge that urgently needs to be addressed in clinical practice.

### SUMMARY OF INVENTION

In order to further develop a novel 2-(1-hydroxypentyl)benzoate salt to overcome the technical issues existing in the prior art, the present invention provides a novel cycloalkylammonium 2-(1-hydroxypentyl)benzoate salt, the preparation and use thereof. The novel cycloalkylammonium 2-(1-hydroxypentyl)benzoate salt provided by the present invention possesses a good solid form and significant pharmacological efficacy.

On the one hand, the present invention provides a cycloalkylammonium 2-(1-hydroxypentyl)benzoate salt of the structure represented by formula (I), or isomers thereof: 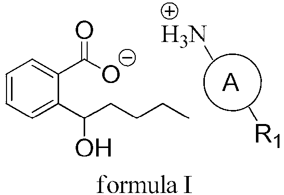 wherein cycle A is selected from adamantane or a 3 to 7-membered cycloalkane, and R₁ is independently 1 to 3 selected from: hydrogen, alkyl, hydroxyl, halogen or haloalkyl.

In some embodiments, The above-mentioned cycle A is selected from adamantane, cyclopropane, cyclobutane, cyclopentane, cyclohexane, cycloheptane, or bicyclo[2.2.1]heptane.

In some embodiments, the above-mentioned R₁ is independently 1 to 3 selected from: hydrogen, C1~C6 alkyl, hydroxyl, halogen, or halo-C1~C6 alkyl.

In some embodiments, the above-mentioned cycle A is selected from adamantane; and/or the above R₁ is independently 1 to 3 selected from: hydrogen, C1~C6 alkyl, hydroxyl, halogen, or halo-C1~C6 alkyl.

In some embodiments, the above-mentioned C1~C6 alkyl includes, but is not limited to, methyl, ethyl, propyl, isopropyl, n-butyl, isobutyl, tert-butyl, n-pentyl, or n-hexyl; and/or the above-mentioned halogen includes, but is not limited to, fluorine, chlorine, or bromine; and/or the above-mentioned halo-C1~C6 alkyl includes, but is not limited to, trifluoromethyl or difluoromethyl.

In some embodiments, the above-mentioned cycloalkylammonium 2-(1-hydroxypentyl)benzoate salt or isomers thereof include the following compounds:

| **NO.** | **Structural formula** | **Chemical Name** |
|---|---|---|
| Compound 1 | 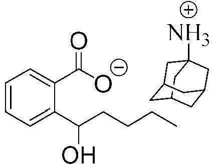 | amantadine |
| | | 2-(1-hydroxypentyl)benzoate salt |
| Compound 2 | 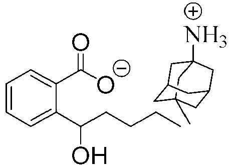 | 3-methyladamantan-1-amine |
| | | 2-(1-hydroxypentyl)benzoate salt |
| Compound 3 | 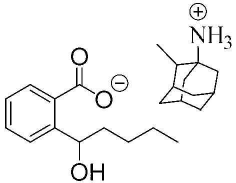 | 2-methyladamantan-1-amine |
| | | 2-(1-hydroxypentyl)benzoate salt |
| Compound 4 | 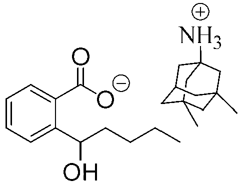 | 3,5-dimethyladamantane-1-amine |
| | | 2-(1-hydroxypentyl)benzoate salt |
| Compound 5 | 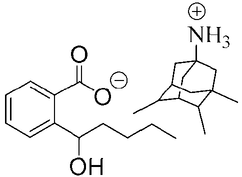 | 3,4,6-trimethyladamantane-1-amine |
| | | 2-(1-hydroxypentyl)benzoate salt |
| Compound 6 | 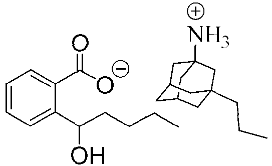 | 3-n-propyladamantane-1-amine |
| | | 2-(1-hydroxypentyl)benzoate salt |
| Compound 7 | 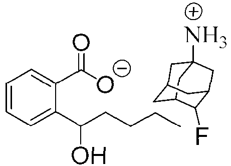 | 4-fluoroadamantane-1-amine |
| | | 2-(1-hydroxypentyl)benzoate salt |
| Compound 8 | 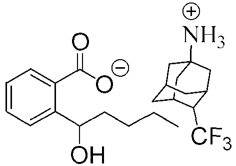 | 4-trifluoromethyladamantan-1-amine |
| | | 2-(1-hydroxypentyl)benzoate salt |
| Compound 9 | 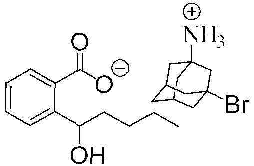 | 3-bromoadamantan-1-amine |
| | | 2-(1-hydroxypentyl)benzoate salt |
| Compound 10 | 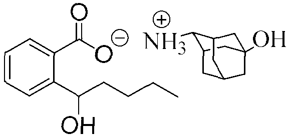 | trans-1-hydroxyadamantan-4-amine |
| | | 2-(1-hydroxypentyl)benzoate salt |
| Compound 11 | 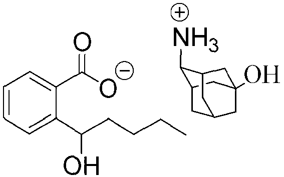 | cis-1-hydroxyadamantan-4-amine |
| | | 2-(1-hydroxypentyl)benzoate salt |
| Compound 12 | 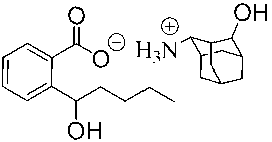 | 4-hydroxyadamantan-2-amine |
| | | 2-(1-hydroxypentyl)benzoate salt |
| Compound 13 | 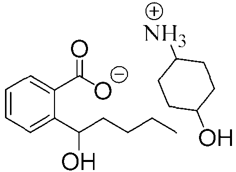 | 4-hydroxycyclohexan-1-amine |
| | | 2-(1-hydroxypentyl)benzoate salt |
| Compound 14 | 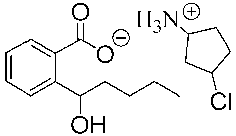 | 3-chlorocyclopentan-1-amine |
| | | 2-(1-hydroxypentyl)benzoate salt |
| Compound 15 | 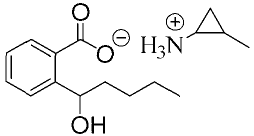 | 2-methylcyclopropane-1-amine |
| | | 2-(1-hydroxypentyl)benzoate salt |
| Compound 16 | 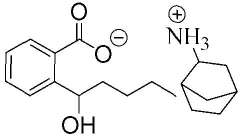 | Bicyclo[2,2,1]heptane-2-amine |
| | | 2-(1-hydroxypentyl)benzoate salt |
| Compound 17 | 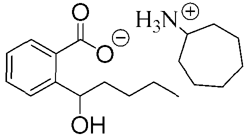 | Cycloheptanamine |
| | | 2-(1-hydroxypentyl)benzoate salt |
| Compound 18 | 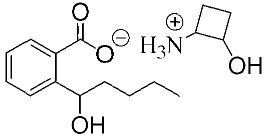 | 2-hydroxycyclobutan-1-amine |
| | | 2-(1-hydroxypentyl)benzoate salt |

When the structure and nomenclature of a compound according to the present invention conflict and cannot be reasonably explained, the structure prevails.

In some embodiments, the hydrogen in the above-mentioned compound may be replaced by one or more deuteriums.

On the other hand, the present invention provides a method of preparing the above-mentioned cycloalkylammonium 2-(1-hydroxypentyl)benzoate salt or isomers thereof, comprising the following steps:

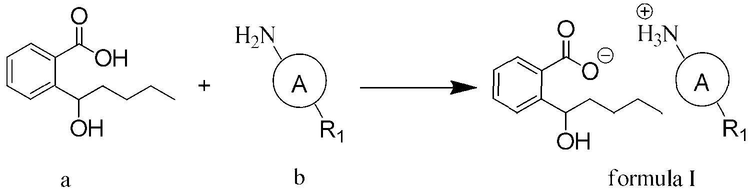

Compound a and compound b undergo a salt formation reaction in the presence of an organic solvent, yield a compound of Formula I;
wherein cycle A, R₁ are defined as any of the corresponding definitions above.

In some embodiments, said organic solvent comprises, but is not limited to, ether-based solvent or ester-based solvent; preferably, said ether-based solvent comprises, but is not limited to, ether or methyl tert-butyl ether; said ester-based solvent comprises, but is not limited to, ethyl acetate, isopropyl acetate, or butyl acetate; and/or the salt is formed at fa temperature between 0-50°C, preferably 10-30°C.

In a third aspect, the present invention also provides a pharmaceutical composition comprising any of the foregoing cycloalkylammonium 2-(1-hydroxypentyl)benzoate salts or isomers thereof, together with a pharmaceutically acceptable carrier.

The term "pharmaceutically acceptable carrier" according to the present invention refers to a diluent, adjuvant, excipient, or vehicle that is administered with the active ingredient and that, within the bounds of reasonable medical judgment, is suitable for contact with the tissues of humans or other animals without undue toxicity, irritation, allergic response, or other problems or complications beyond those commensurate with a reasonable benefit-risk ratio.

In some embodiments, the above-mentioned pharmaceutical compositions include, but are not limited to, tablets, capsules, large-volume injections, small-volume injections, lyophilized powders, or granules.

In a fourth aspect, the present invention also provides the use of the above-mentioned cycloalkylammonium 2-(1-hydroxypentyl)benzoate salt or isomers thereof in the preparation of a medicament for the treatment and/or prevention of ischemic cardiovascular and cerebrovascular diseases.

In some embodiments, said ischemic cardiac and cerebrovascular diseases include, but are not limited to, mild, moderate, or severe acute ischemic stroke, cerebral thrombosis, cerebral embolism, coronary artery disease, angina pectoris, or myocardial infarction; preferably, moderate or severe acute ischemic stroke; and more preferably improvement of neurological deficits in patients with acute ischemic stroke.

Beneficial effects: The cycloalkylammonium 2-(1-hydroxypentyl)benzoate salt according to the present invention exhibts at least of the following technical effects: (1) a good solid form and a simple preparation method, suitable for industrialized scaled-up production; (2) strong anti-platelet aggregation effect, strong effect in improving neurological symptoms and suppression of cerebral infarction area, demonstrating that the compound of the present invention possesses the ability to prevent and/or treat heart and brain arterial blockage, improve the cardiac and cerebral microcirculation and other pharmacological effects, suitable for use in the prevention and/or treatment of cardiac and cerebral ischemic diseases; (3) a wide therapeutic window, ensuring medication safety; and (4) good solubility and high stability, suitable for pharmaceutical development, especially with the strong potential for formulation as an intravenous injection.

### DESCRIPTION OF EMBODIMENTS

Hereinafter, the technical solution according to the present invention will be further described by way of examples. It will be understood by those skilled in the art that the following embodiments are provided for illustration only and are not intended to limit the scope of the invention. Unless otherwise specified, techniques or conditions not expressly set out in the embodiments may be carried out by those skilled in the art with reference to the literature in the art or the product manuals. If no specific manufacturers are specified in the examples, the reagents and instruments are commercially available products.

The structures of the compounds were determined by nuclear magnetic resonance (1H NMR) or liquid mass spectrometry (LC-MS).

The liquid chromatography-mass spectrometry (LC-MS) was an Agilent G6120B equipped with an Agilent 1260; the nuclear magnetic resonance (1H NMR) instruments were a Bruker AVANCE-400 or a Bruker AVANCE-800; and the nuclear magnetic resonance (1H NMR) shifts (δ) were given in parts per million (ppm), with DMSO as the solvent and TMS as the internal standard.

The term "room temperature" according to the present invention refers to a temperature between 10 and 30°C. Unless otherwise specified, the reagents and compounds used in the present invention are commercially available.

The term "xxx group" as used in the test examples according to the present invention indicates that the drug used in that group is "xxx". By way of example, "Compound 1 group" means that the drug used in that group is "Compound 1".

### Example 1: Preparation of amantadine 2-(1-hydroxypentyl) benzoate salt

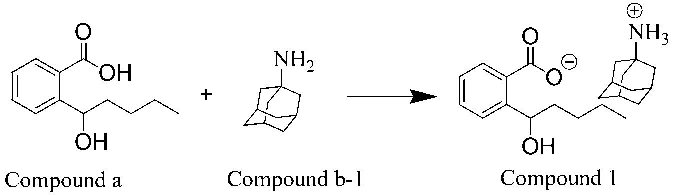

Preparation method: 10.00 g of compound a (2-(1-hydroxypentyl) benzoic acid) was dissolved in 40 ml of methyl tert-butyl ether. The reaction solution was stirred at room temperature, and 7.26 g of amantadine (compound b-1) was dissolved in 30 ml of methyl tert-butyl ether. The amantadine solution in methyl tert-butyl ether was added dropwise to the reaction system at room temperature. After the dropwise addition, the reaction system was cooled to -5 to 20°C, and suction filtration was performed. The resulting solid was dried in a vacuum drying oven to obtain the title compound 16.50 g, with yield 90%, purity 99.2%.

ESI-MS: positive ion mode m/z = 152.1 (amantadine + H) ⁺, negative ion mode m/z = 207.1 (compound a-H) ⁻.

¹H NMR (400 MHz, DMSO-d6) : 8.17 (3 H, s), 7.62 - 7.64 (1 H, m), 7.13 - 7.23 (3 H, m), 4.53 - 4.56(1 H, m), 2.05 (3 H, s), 1.81 (6 H, d), 1.63 - 1.73 (5 H, m), 1.55 - 1.60 (3 H, m), 1.30 - 1.39 (1 H, m), 1.20 - 1.29 (2 H, m), 1.09 - 1.18 (1 H, m), 0.82 (3 H, t).

### Example 2: Preparation of 3-methyladamantan-1-amine 2-(1-hydroxypentyl)benzoate salt

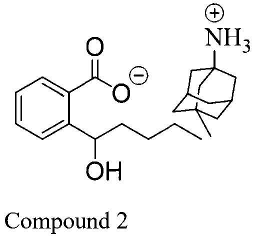

The preparation method was the same as in example 1, except that the adamantane was replaced with an equimolar amount of 3-methyladamantan-1-amine, and the organic solvent was replaced with ethyl acetate, resulting in 15.25 g of the solid title compound with 85% yield and 99.3% purity.

ESI-MS: positive ion mode m/z = 166.2 (3-methyladamantan-1-amine + H) ⁺, negative ion mode m/z = 207.1 (compound a-H) ⁻.

¹H NMR (400 MHz, DMSO-d6) : 8.17 (3 H, s), 7.62 - 7.64 (1 H, m), 7.13 - 7.23 (3 H, m), 4.53 - 4.56(1 H, m), 2.05 (3 H, s), 1.81 (6 H, d), 1.63 - 1.73 (5 H, m), 1.55 - 1.63 (2 H, m), 1.09 - 1.39 (4 H, m), 0.81 - 0.83 (6 H, m).

### Example 3: Preparation of 2-methyladamantan-1-amine 2-(1-hydroxypentyl)benzoate salt

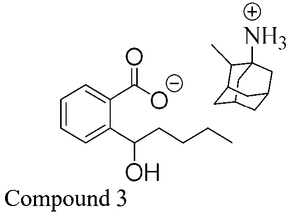

The preparation method was the same as in example 1, except that amantadine was replaced with an equimolar amount of 2-methyladamantan-1-amine, and the organic solvent was replaced with butyl acetate, resulting in 15.73 g of the solid title compound with 88% yield and 99.3% purity.

ESI-MS: positive ion mode m/z = 166.2 (2-methyladamantan-1-amine + H) ⁺, negative ion mode m/z = 207.1 (compound a-H) ⁻.

¹H NMR (400 MHz, DMSO-d6) : 8.17 (3 H, s), 7.62 - 7.64 (1 H, m), 7.13 - 7.23 (3 H, m), 4.53 - 4.56(1 H, m), 2.05 (3 H, s), 1.81 (6 H, d), 1.63 - 1.73 (5 H, m), 1.55 - 1.63 (2 H, m), 1.09 - 1.39 (4 H, m), 0.81 - 0.83 (6 H, m).

### Example 4: Preparation of 3,5-dimethyladamantan-1-amine 2-(1-hydroxypentyl)benzoate salt

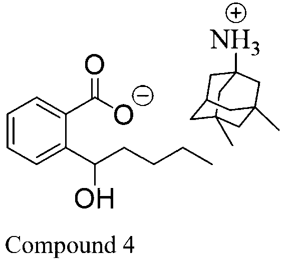

The preparation method was the same as in example 1, except that amantadine was replaced with an equimolar amount of 3,5-dimethyladamantan-1-amine, resulting in 15.82 g of the solid title compound with 85% yield and 99.5% purity.

ESI-MS: positive ion mode m/z = 180.1 (3,5-dimethyladamantan-1-amine + H) ⁺, negative ion mode m/z = 207.1 (compound a-H)⁻.

¹H NMR (400 MHz, DMSO-d6) : 8.16 (3 H, s), 7.62 - 7.64 (1 H, m), 7.13 - 7.23 (3 H, m), 4.53 - 4.56(1 H, m), 1.80 - 2.07 (9 H, m), 1.62 - 1.73 (4 H, m), 1.54 - 1.60 (2 H, m), 1.07 - 1.38 (4 H, m), 0.81 -0.84 (9 H, m).

### Example 5: Preparation of 3,4,6-trimethyladamantan-1-amine 2-(1-hydroxypentyl)benzoate salt

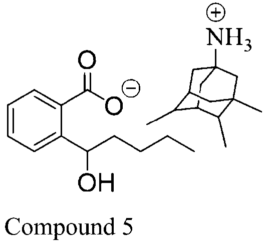

The preparation method was the same as in example 1, except that amantadine was replaced with an equimolar amount of 3,4,6-trimethyladamantan-1-amine, resulting in 15.63 g of the solid title compound with 84% yield and 99.0% purity.

ESI-MS: positive ion mode m/z = 194.3 (3,4,6-trimethyladamantan-1-amine + H) ⁺, negative ion mode m/z = 207.1 (compound a-H)⁻.

¹H NMR (400 MHz, DMSO-d6) : 8.18 (3 H, s), 7.62 - 7.64 (1 H, m), 7.13 - 7.23 (3 H, m), 4.53 - 4.56(1 H, m), 1.81 - 2.07 (9 H, m), 1.62 - 1.73 (4 H, m), 1.07 - 1.61 (5 H, m), 0.81 -0.84 (12 H, m).

### Example 6: Preparation of 3-n-propyladamantan-1-amine 2-(1-hydroxypentyl)benzoate salt

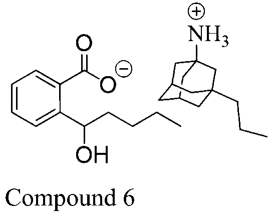

The preparation method was the same as in example 1, except that amantadine was replaced with an equimolar amount of 3-n-propyladamantane-1-amine, resulting in 16.58 g of the solid title compound with 86% yield and 99.1% purity.

ESI-MS: positive ion mode m/z = 194.3 (3-n-propyladamantan-1-amine + H) ⁺, negative ion mode m/z = 207.1 (compound a-H)⁻.

¹H NMR (400 MHz, DMSO-d6) : 8.18 (3 H, s), 7.62 - 7.64 (1 H, m), 7.13 - 7.23 (3 H, m), 4.53 - 4.56(1 H, m), 1.79 - 2.07 (9 H, m), 1.63 - 1.73 (4 H, m), 1.43 - 1.62 (7 H, m), 1.07 - 1.38 (4 H, m), 0.80 -0.85 (6 H, m).

### Example 7: Preparation of 4-fluoroadamantan-1-amine 2-(1-hydroxypentyl)benzoate salt

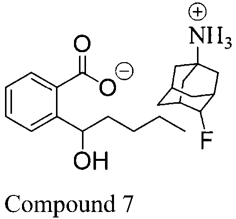

The preparation method was the same as in example 1, except that amantadine was replaced with an equimolar amount of 4-fluoroadamantan-1-amine, resulting in 16.13 g of the solid title compound with 89% yield and 99.3% purity.

ESI-MS: positive ion mode m/z = 170.2 (4-fluoroadamantan-1-amine + H) ⁺, negative ion mode m/z = 207.1 (compound a-H) ⁻.

¹H NMR (400 MHz, DMSO-d6) : 8.17 (3 H, s), 7.62 - 7.64 (1 H, m), 7.13 - 7.23 (3 H, m), 4.53 - 4.56(1 H, m), 2.06 (3 H, s), 1.07 - 1.83 (17 H, m), 0.82 (3 H, t).

### Example 8: Preparation of 4-trifluoromethyladamantan-1-amine 2-(1-hydroxypentyl)benzoate salt

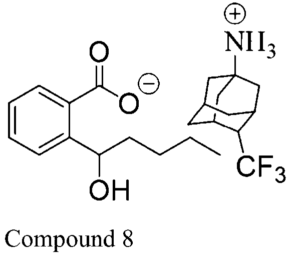

The preparation method was the same as in example 1, except that amantadine was replaced with an equimolar amount of 4-trifluoromethyladamantan-1-amine, resulting in 17.86 g of the solid title compound with 87% yield and 99.2% purity.

ESI-MS: positive ion mode m/z = 220.2 (4-trifluoromethyladamantan-1-amine + H) ⁺, negative ion mode m/z = 207.1 (compound a-H)⁻.

¹H NMR (400 MHz, DMSO-d6) : 8.17 (3 H, s), 7.62 - 7.64 (1 H, m), 7.13 - 7.23 (3 H, m), 4.53 - 4.56(1 H, m), 2.06 (3 H, s), 1.07 - 1.83 (17 H, m), 0.82 (3 H, t).

### Example 9: Preparation of 3-bromoadamantan-1-amine 2-(1-hydroxypentyl)benzoate salt

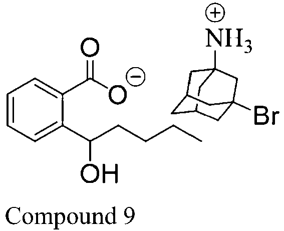

The preparation method was the same as in example 1, except that amantadine was replaced with an equimolar amount of 3-bromoadamantan-1-amine, resulting in 18.95 g of the solid title compound with 90% yield and 99.5% purity.

ESI-MS: positive ion mode m/z = 231.1 (3-bromoadamantan-1-amine + H) ⁺, negative ion mode m/z = 207.1 (compound a-H)⁻.

¹H NMR (400 MHz, DMSO-d6) : 8.17 (3 H, s), 7.62 - 7.64 (1 H, m), 7.13 - 7.23 (3 H, m), 4.53 - 4.56(1 H, m), 2.05 (3 H, s), 1.09 - 1.81 (17 H, m), 0.82 (3 H, t).

### Example 10: Preparation of trans-1-hydroxyadamantan-4-amine 2-(1-hydroxypentyl)benzoate salt

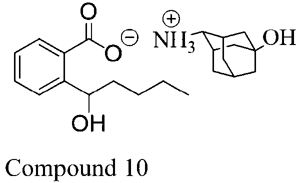

The preparation method was the same as in example 1, except that amantadine was replaced with an equimolar amount of trans-1-hydroxyadamantan-4-amine, resulting in 16.59 g of the solid title compound with 92% yield and 99.5% purity.

ESI-MS: positive ion mode m/z = 168.1 (trans-1-hydroxyadamantan-4-amine + H) ⁺, negative ion mode m/z = 207.1 (compound a-H)⁻.

¹H NMR (400 MHz, DMSO-d6) : 8.23 (3 H, s), 7.61 - 7.63 (1 H, m), 7.13 - 7.21 (3 H, m), 4.53 - 4.56(1 H, m), 2.11 - 0.91 (20H, m), 0.81 (3 H, t).

### Example 11: Preparation of cis-1-hydroxyadamantan-4-amine 2-(1-hydroxypentyl)benzoate salt

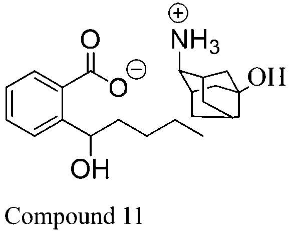

The preparation method was the same as in example 1, except that amantadine was replaced with an equimolar amount of cis-1-hydroxyadamantan-4-amine, resulting in 16.59 g of the solid title compound with 92% yield and 99.0% purity.

ESI-MS: positive ion mode m/z = 168.1 (cis-1-hydroxyadamantan-4-amine + H) ⁺, negative ion mode m/z = 207.1 (compound a-H)⁻.

¹H NMR (400 MHz, DMSO-d6) : 8.23 (3 H, s), 7.61 - 7.63 (1 H, m), 7.13 - 7.21 (3 H, m), 4.53 - 4.56(1 H, m), 2.09 - 0.91 (20H, m), 0.81 (3 H, t).

### Example 12: Preparation of 4-hydroxyadamantan-2-amine 2-(1-hydroxypentyl)benzoate salt

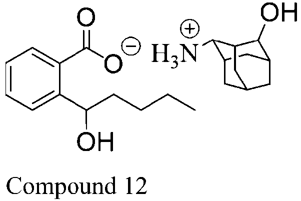

The preparation method was the same as in example 1, except that amantadine was replaced with an equimolar amount of 4-hydroxyadamantan-2-amine, resulting in 16.23 g of the solid title compound with 90% yield and 99.2% purity.

ESI-MS: positive ion mode m/z = 168.1 (4-hydroxyadamantan-2-amine + H) ⁺, negative ion mode m/z = 207.1 (compound a-H)⁻.

¹H NMR (400 MHz, DMSO-d6) : 8.23 (3 H, s), 7.61 - 7.63 (1 H, m), 7.13 - 7.21 (3 H, m), 4.53 - 4.56(1 H, m), 2.08 - 0.90 (20H, m), 0.82 (3 H, t).

### Example 13: Preparation of 4-hydroxycyclohexan-1-amine 2-(1-hydroxypentyl)benzoate salt

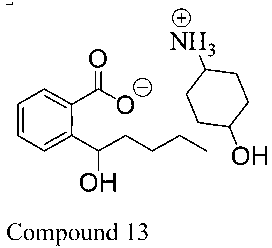

The preparation method was the same as in example 1, except that amantadine was replaced with an equimolar amount of 4-hydroxycyclohexan-1-amine, resulting 14.74 g of the solid title compound with 95% yield and 99.2% purity.

ESI-MS: positive ion mode m/z = 116.1 (4-hydroxycyclohexan-1-amine + H) ⁺, negative ion mode m/z = 207.1 (compound a-H) ⁻.

¹H NMR (400 MHz, DMSO-d6) : 8.23 (3 H, s), 7.61 - 7.63 (1 H, m), 7.13 - 7.21 (3 H, m), 4.53 - 4.56(1 H, m), 3.51 - 3.54 (2H, m), 1.25 - 2.37 (14H, m) 0.81 (3 H, t).

### Example 14: Preparation of 3-chlorocyclopentan-1-amine 2-(1-hydroxypentyl)benzoate salt

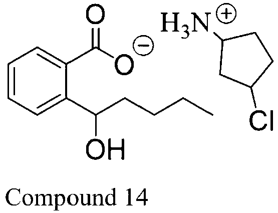

The preparation method was the same as in example 1, except that amantadine was replaced with an equimolar amount of 3-chlorocyclopentan-1-amine, resulting 14.01 g of the solid title compound with 89% yield and 99.0% purity.

ESI-MS: positive ion mode m/z = 120.1 (3-chlorocyclopentan-1-amine + H) ⁺, negative ion mode m/z = 207.1 (compound a-H) ⁻.

¹H NMR (400 MHz, DMSO-d6) : 8.23 (3 H, s), 7.61 - 7.63 (1 H, m), 7.13 - 7.21 (3 H, m), 4.53 - 4.56(1 H, m), 3.53 - 3.57 (2H, m), 1.23 - 2.42 (12H, m) 0.81 (3 H, t).

### Example 15: Preparation of 2-methylcyclopropan-1-amine 2-(1-hydroxypentyl)benzoate salt

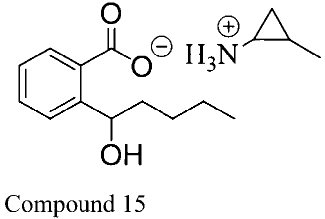

The preparation method was the same as in example 1, except that amantadine was replaced with an equimolar amount of 2-methylcyclopropan-1-amine, resulting in 11.54 g of the solid title compound with 86% yield and 98.8% purity.

ESI-MS: positive ion mode m/z = 72.1 (2-methylcyclopropan-1-amine + H) ⁺, negative ion mode m/z = 207.1 (compound a-H) ⁻.

¹H NMR (400 MHz, DMSO-d6) : 8.23 (3 H, s), 7.61 - 7.63 (1 H, m), 7.13 - 7.21 (3 H, m), 4.53 - 4.56(1 H, m), 3.43 - 3.55 (2H, m), 1.23 - 2.42 (6H, m), 0.81 -0.84 (6 H, m), 0.37 -0.62 (2 H, m).

### Example 16: Preparation of bicyclo[2,2,1]heptan-2-amine 2-(1-hydroxypentyl)benzoate salt

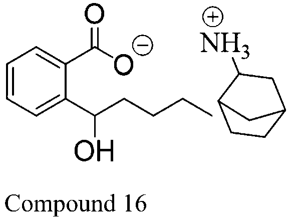

The preparation method was the same as in example 1, except that amantadine was replaced with an equimolar amount of bicyclo[2,2,1]heptan-2-amine, resulting 13.87 g of the solid title compound with 90% yield and 98.2% purity.

ESI-MS: positive ion mode m/z = 112.2 (bicyclo[2,2,1]heptan-2-amine + H) ⁺, negative ion mode m/z = 207.1 (compound a-H) ⁻.

¹H NMR (400 MHz, DMSO-d6) : 8.23 (3 H, s), 7.61 - 7.63 (1 H, m), 7.13 - 7.21 (3 H, m), 4.53 - 4.56(1 H, m), 3.52 - 3.56 (1H, m), 1.25 - 2.37 (16H, m) 0.81 (3 H, t).

### Example 17: Preparation of cycloheptanamine 2-(1-hydroxypentyl)benzoate salt

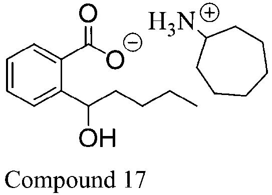

The preparation method was the same as in example 1, except that amantadine was replaced with an equimolar amount of cycloheptanamine, resulting 12.87 g of the solid title compound with 83% yield and 99.0% purity.

ESI-MS: positive ion mode m/z = 114.2 (cycloheptanamine + H) ⁺, negative ion mode m/z = 207.1 (compound a-H)⁻.

¹H NMR (400 MHz, DMSO-d6) : 8.23 (3 H, s), 7.61 - 7.63 (1 H, m), 7.13 - 7.21 (3 H, m), 4.53 - 4.56(1 H, m), 3.52 - 3.56 (1H, m), 1.25 - 2.37 (18H, m) 0.82 (3 H, t).

### Example 18: Preparation of 2-hydroxycyclobutan-1-amine 2-(1-hydroxypentyl)benzoate salt

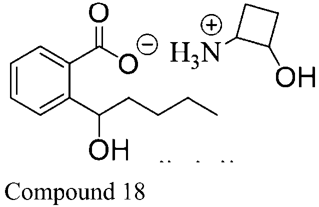

The preparation method was the same as in example 1, except that amantadine was replaced with an equimolar amount of 2-hydroxycyclobutan-1-amine, resulting 12.06 g of the solid title compound with 85% yield and 99.2% purity.

ESI-MS: positive ion mode m/z = 88.1 (2-hydroxycyclobutan-1-amine + H) ⁺, negative ion mode m/z = 207.1 (compound a-H)⁻.

¹H NMR (400 MHz, DMSO-d6) : 8.23 (3 H, s), 7.61 - 7.63 (1 H, m), 7.13 - 7.21 (3 H, m), 4.53 - 4.56(1 H, m), 3.50 - 3.55 (2H, m), 1.24 - 2.36 (10H, m) 0.812 (3 H, t).

**Comparative Example 1:** 2-(1-hydroxypentyl)benzoic acid potassium salt (abbreviated as "potassium salt"), prepared according to patent CN1382682A, with of purity of 99.7%.

**Comparative Example 2:** 2-(1-hydroxypentyl)benzoic acid tert-butylamine salt (abbreviated as "tert-butylamine salt"), prepared according to patent CN1523003A, with of purity of 99.8%.

**Comparative Example 3:** Preparation of 2-(1-hydroxypentyl)benzoic acid diisopropylamine salt (abbreviated as "diisopropylamine salt")

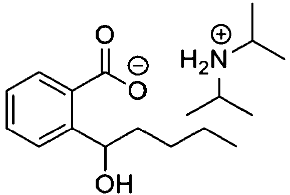

It was prepared according to the method of Example 1, except that amantadine was replaced with an equimolar amount of diisopropylamine, resulting 12.2 g of the solid title compound with 82% yield and 99.7% purity.

ESI-MS: positive ion mode m/z = 102.3 (diisopropylamine + H) ⁺, negative ion mode m/z = 207.1 (compound a-H)⁻.

¹H NMR (400 MHz, DMSO-d6) : 8.15 (2 H, s), 7.61 - 7.63 (1 H, m), 7.13 - 7.21 (3 H, m), 4.53 - 4.56(1 H, m), 3.62 - 3.78 (2H, m), 1.68 (12, d)1.24 - 1.55 (6H, m) 0.812 (3 H, t).

### Test Example 1: Evaluation of short-term acute pharmacological efficacity in a rat ischemia-reperfusion stroke model

### (I) Neurological function behavioral scoring

**1. Groups and Drug Administration:** Rats were randomly divided into 8 groups, i.e., model group, sham operation group, butylphthalide (CAS:6066-49-5) group, the compound 1 group, the compound 4 group, the compound 10 group, the potassium salt (comparative example 1 compound), and adamandine (CAS:768-94-5) group. Administration was initiated upon reperfusion after 2 hours of ischemia. The model and the sham operation group were given an equal volume 5% glucose injection solution via tail vein injection, while the other groups were injected with the corresponding drugs via tail vein, each with a single dose.
**2. Performing modeling surgery:** The rat MCAO model was established with reference to the Longa intraluminal filament occlusion method. Nylon monofilament was cut into 2 cm lengths, the tip was dipped into a tiny amount of molten paraffin, after cooling, the ends of the nylon threads were completely wrapped for later use. The rats were completely anesthetized with 3% sodium pentobarbital (30 mg/kg), and placed supine, and secured on a surgical board. The cervical skin was prepared and disinfected, a 2-cm mid line incision was made. The left common carotid artery was bluntly isolated, and the external and internal carotid arteries were dissected free along the common carotid artery. The distal ends of the common carotid artery and the external carotid artery were ligated, and the internal carotid artery was occluded using hemostatic forceps. A small arteriotomy was made in the common carotid artery and the prepared nylon monofilament was inserted through the opening. The wax-coated tip was inserted into the common carotid artery and advanced into the internal carotid artery, the hemostat was released, and the advancement of the wax tip was stopped when it was 13-16 mm from the opening. The filament was secured, and the cervical incision was closed. After 2 h of occlusion, rats were anesthetized and the filament was withdrawn. In the sham operation group, rats were completely anesthetized with only 3% sodium pentobarbital. After cervical skin preparation and incision, the common carotid artery was bluntly isolated, and the wound immediately closed, without filament insertion.
**3. Neurological deficit scoring:** scoring time: before modeling, 2 h after ischemia, and 24 h post-surgery, all surviving animals were scored in a blinded manner using a modified neurological deficit score. The scoring criteria of Longa's method were as follows: 0: normal neurological function; 1: mild neurological deficit: when suspended by the tail, the animal's left forelimb was flexed; 2: moderate neurological deficit: when placed on a smooth surface, the animal circles to the left side while walking; 3: moderate neurological deficit: at rest, the animal leans to the left side; 4: depressed consciousness with no spontaneous limb movements; 5: unresponsive to stimuli or death.
**4. Result analysis:** The t-test was used to compare the behavioral test scores between groups. The results are shown in the table below.

**Table 1 Statistical results of the improvement of neurological symptoms of MCAO rats by each compound**

| **Group** | **Dose (mmol/kg)** | **Number of animals (n)** | **Score** | |
|---|---|---|---|---|
| | | | **2h** | **24h** |
| Model group | - | 10 | 2.6±1.26 | 3.2±1.23 |
| Sham operation group | - | 10 | 0±0.00 | 0±0.00 |
| Butylphthalide group | 0.053 | 10 | 2.8±1.03 | 2.10±0.99* |
| Compound 1 group | 0.016 | 10 | 2.8±1.14 | 1.60±0.84** |
| Compound 4 group | 0.016 | 10 | 2.7±1.16 | 1.50±0.53** |
| Compound 10 group | 0.016 | 10 | 2.9±1.52 | 1.61±1.07** |
| Potassium salt group | 0.016 | 10 | 2.9±0.99 | 1.90±0.74* |
| Amantadine group | 0.016 | 10 | 2.5±1.08 | 3.00±0.82 |

| | | | | |
|---|---|---|---|---|
| Note: Compared with the model group, **P<0.01, *P<0.05. | | | | |

The results indicated that no abnormal changes in behavior were observed in the sham-operated group. The rats in the model group, butylphthalide group, compound 1 group, compound 4 group, compound 10 group, potassium salt group, and amantadine group all exhibited hemiparesis-like symptoms at 2 h after surgery, mainly manifested as adduction of the contralateral forelimb with shoulder internal rotation and reduced forelimb muscle tone on the contralateral side of the operatio. Compared with the model group, the neurological symptoms of rats in the butylphthalide group and potassium salt group were significantly improved at 24 h postoperatively (P<0.05); the neurological symptoms of rats in the compound 1 group, compound 4 group, and compound 10 group were highly significant (P<0.01); and there was no significant improvement in the neurological symptoms of rats in the amantadine group (P>0.05). These results demonstrate that the compounds according to the present invention showed greater improvement in neurological deficits in MCAO rats than the potassium salt.

### (ii) Cerebral infarction volume

24 hours after MCAO ischemia, the surviving rats of each group were dissected, pre-cooled PBS was perfused into the heart. The brain tissue was taken and stained with 1% Tetrazolium Red (TTC). The infarct volume percentage was determined. Infarct volume percentage = (right infarct volume/right brain volume) × 100%. The t-test was used to compare the cerebral infarct volume percentage in individual groups. The results are shown in the table below.

**Table 2 Statistical results of cerebral infarct volume of MCAO rats in each compound group**

| **Group** | **Dose (mmol/kg)** | **Number of animals (n)** | **Percentage of cerebral infarction volume (%)** |
|---|---|---|---|
| Model group | - | 10 | 40.3±8.73 |
| Sham operation group | - | 10 | 0±0.00 |
| Butylphthalide group | 0.053 | 10 | 31.0±7.50* |
| Compound 1 group | 0.016 | 10 | 25.7±5.29** |
| Compound 4 group | 0.016 | 10 | 25.1±4.41** |
| Compound 10 group | 0.016 | 10 | 25.8±4.52** |
| Potassium salt group | 0.016 | 10 | 29.7±5.72** |
| Amantadine group | 0.016 | 10 | 35.1±4.56 |

| | | | |
|---|---|---|---|
| Note: Compared with the model group, **P<0.01, *P<0.05. | | | |

The results indicated that rats in the model group showed evident infarct areas after 24h of middle cerebral artery infarction, whereas no changes were observed in sham-operated controls. Compared with the model group, the extent of cerebral infarction was significantly reduced in the rats in the butylphthalide group (P<0.05); the extent of cerebral infarction was significantly reduced in the rats in the compound 1 group, the compound 4 group, the compound 10 group, and the potassium salt group (P<0.01); and there was no significant inhibitory effect on any of the rats in the amantadine group (P>0.05). It was demonstrated that at equimolar doses, the compounds according to the present invention inhibited the extent of cerebral infarction in MCAO rats more strongly than butylphthalide and potassium salt.

### Test example 2: Effect on platelet aggregation in rats

Rats were randomly divided into 14 groups: i.e. solvent control group, aspirin group, butylphthalide group, compound 1 group, compound 3 group, compound 4 group, compound 6 group, compound 7 group, compound 10 group, compound 13 group, compound 15 group, compound 16 group, potassium salt group, and N'N dibenzylethylenediamine salt group. After oral gavage administration for 3 hours on the same day, blood was collected from the abdominal aorta to obtain platelet-rich plasma from each group. Platelet aggregation was induced by 20µM ADP, and the maximum platelet aggregation rate in platelet-rich plasma was determined by platelet aggregometer, to evaluate and compare the effects of platelet aggregation in rats.

**Table 3 Statistical results of platelet aggregation effect of each compound**

| **Group** | **Route of administration** | **Number of animals (n)** | **Dose (mg/kg)** | **Maximum platelet aggregation rate (%)** |
|---|---|---|---|---|
| Solvent control group | ig | 10 | - | 67.84±9.73 |
| Aspirin group | ig | 10 | 100 | 27.00±6.48** |
| Butylphthalide group | ig | 10 | 200 | 57.50±9.05* |
| Compound 1 group | ig | 10 | 200 | 34.60±9.57** |
| Compound 3 group | ig | 10 | 200 | 37.50±9.42** |
| Compound 4 group | ig | 10 | 200 | 38.10±6.24** |
| Compound 6 group | ig | 10 | 200 | 40.30±12.26** |
| Compound 7 group | ig | 10 | 200 | 37.90±12.60** |
| Compound 10 group | ig | 10 | 200 | 38.20±12.25** |
| Compound 13 group | ig | 10 | 200 | 42.10±5.99** |
| Compound 15 group | ig | 10 | 200 | 41.10±6.45** |
| Compound 16 group | ig | 10 | 200 | 44.10±5.15** |
| Potassium salt group | ig | 10 | 200 | 48.50±8.29** |
| N'N-dibenzylethylenediamine salt | ig | 10 | 200 | 46.6±10.78** |

| | | | | |
|---|---|---|---|---|
| Note: Compared with the solvent control group, *P<0.05, **P<0.01. N'N dibenzylethylenediamine salt was prepared according to patent CN1523003A with 99.3% purity. | | | | |

The results showed that, at the same dose, the platelet aggregation rate of the rats in the butylphthalide group was significantly different (P<0.05) from that in the solvent control group; the platelet aggregation rate of rats in the aspirin group, the compounds of each example compound group, the potassium salt group, and the N'N dibenzylethylenediamine salt group were all highly significantly different (P<0.01) from that of the solvent control group. Among these, the platelet aggregation rates of the rats in the compound groups of each embodiment were much lower than those in the butylphthalide group, the potassium salt group and the N'N dibenzylethylenediamine salt group, demonstrating that the compounds according to the present invention exhibit superior antiplatelet aggregation effects.

### Test example 3: Repeated Administration Toxicity Test in Rats

Healthy adult SD rats were selected and randomly divided into 8 groups, with 10 rats per group, half male and half female, classified as: blank group, solvent control group, low-dose compound 1 group, medium-dose compound 1 group, high-dose compound 1 group, low-dose compound 4 group, medium-dose compound 4 group, and high-dose compound 4 group. The blank group was intravenously injected via the tail vein with an equal volume of 5% glucose solution, the solvent control group was intravenously injected via the tail vein with an equal volume of the solvent used to prepare the drug, and the treatment group was intravenously injected via the tail vein with the corresponding concentration of the drug, all once daily for 14 consecutive days. The rats' toxic reactions (such as irritation to the tail vein) were observed and recorded daily, and the mortality rate in each group was counted and statistically analyzed.

**Table 4 Toxicity test results of each compound**

| **Group** | **Number of animals per group (n)** | **Dosage (mg/kg)** | **Mortality rate (%)** |
|---|---|---|---|
| Blank Group | 10 | - | 0 |
| Solvent control group | 10 | - | 0 |
| Low-dose compound 1 group | 10 | 15 | 0 |
| Low-dose compound 4 group | 10 | 15 | 0 |
| Medium-dose compound 1 group | 10 | 45 | 0 |
| Medium-dose compound 4 group | 10 | 45 | 0 |
| High-dose compound 1 group | 10 | 105 | 0 |
| High-dose compound 4 group | 10 | 105 | 0 |

The results indicate that no toxicity events occurred at low, medium, or high doses of the compound of the invention, demonstrating that the compound is pharmaceutically safe with a wide therapeutic window, ensuring the safety of the medication.

### Test example 4: Stability test

Drugs to be tested: compound 1, compound 4, potassium salt (comparative example 1 compound), tert-butylamine salt (comparative example 2 compound), diisopropylamine salt (comparative example 3 compound).

An appropriate amount of each compound to be tested was taken, and the influence factor test was conducted according to the General Chapter 9001 of Part 4 of the 2020 Edition of the Chinese Pharmacopoeia. The appearance of the compounds and the content of the main degradation product, butylphthalide, were detected respectively on day 0 and day 30 for each compound. The detection of butylphthalide was performed using high performance liquid chromatography, with a column packed with octadecylsilane-bonded silica gel, and a mobile phase of methanol-water (65:35).

**Table 5 Results of the high humidity test on the influence factors for each compound**

| **Compound** | **Test conditions** | **Property** | **Decomposition product butylphthalide %** |
|---|---|---|---|
| Compound 1 | 0 day | White powder | 0.114 |
| | RH92.5%, 30 days | White powder | 0.298 |
| Compound 4 | 0 day | White powder | 0.107 |
| | RH92.5%, 30 days | White powder | 0.258 |
| Potassium salt | 0 day | White powder | 0.090 |
| | RH92.5%, 30 days | Partially deliquescent and liquefied | 25.687 |
| Tert-butylamine salt | 0 day | White powder | 0.041 |
| | RH92.5%, 30 days | White powder | 10.214 |
| Diisopropylamine salt | 0 day | White powder | 0.087 |
| | RH92.5%, 30 days | White powder | 8.025 |

The results showed that the compounds according to the present invention exhibited unchanged properties, and the main peak and the content of the primary degradation impurity, butylphthalide, remained essentially unchanged after 30 days under high humidity conditions; Meanwhile, after 30 days of testing under open high-temperature and light exposure, the appearance of compounds 1 and 4 remains unchanged, with minimal variation in the main peak and butylphthalide content. Therefore, the compounds according to the present invention exhibit high stability. Further, the preparation process of the compounds according to the present invention is simple and suitable for industrialized scale-up production.

### Test example 5: Solubility test

Appropriate amounts of compound 1 and compound 4 were taken respectively, and the solubility of each compound in different solvents was determined according to the solubility assay method under the general notices of Part 4 of the 2020 Edition of the Chinese Pharmacopoeia.

**Table 6 Solubility test results of each compound**

| **Compound** | **Medium** | **Sample weight (g)** | **Solvent volume (ml)** | **Solubility** |
|---|---|---|---|---|
| Compound 1 | pH4.5 | 0.01038 | 9.0 | Completely soluble |
| | pH6.8 | 0.01095 | 9.0 | Completely soluble |
| | Water | 0.01041 | 9.0 | Completely soluble |
| Compound 4 | pH4.5 | 0.01022 | 9.0 | Completely soluble |
| | pH6.8 | 0.01034 | 9.0 | Completely soluble |
| | Water | 0.01011 | 9.0 | Completely soluble |

The results indicate that the compounds according to the present invention exhibit good water solubility and are suitable for the preparation of intravenous injection solutions, thus providing more reliable options for clinical treatment.

The above descriptions are merely preferred embodiments according to the present invention. It should be noted that, for those skilled in the art, various modifications to these embodiments can be made without departing from the technical principles according to the present invention, and such modifications should also be considered within the scope of the protection according to the present invention.

## Claims

1. A cycloalkylammonium 2-(1-hydroxypentyl) benzoate salt having the structure represented by formula I, or isomers thereof: 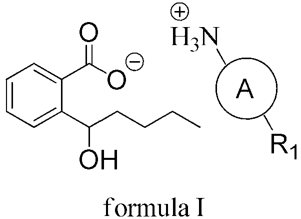 wherein the cycle A is selected from adamantane or a 3 to 7-membered cycloalkane, and R₁ is independently 1 to 3 groups selected from hydrogen, alkyl, hydroxyl, halogen, and haloalkyl.

2. The cycloalkylammonium 2-(1-hydroxypentyl) benzoate salt or isomers thereof according to claim 1, **characterized in that** said cycle A is selected from adamantane; and/or said R₁ is independently 1 to 3 groups selected from hydrogen, C1~C6 alkyl, hydroxyl, halogen, and halo-C1~C6 alkyl.

3. The cycloalkylammonium 2-(1-hydroxypentyl) benzoate salt or isomers thereof according to claim 2, **characterized in that** said C1~C6 alkyl includes, but is not limited to, methyl, ethyl, propyl, isopropyl, n-butyl, isobutyl, tert-butyl, n-pentyl, or n-hexyl; and/or said halogen includes, but is not limited to, fluorine, chlorine, or bromine; and/or said halo-C1~C6 alkyl includes, but is not limited to, trifluoromethyl or difluoromethyl.

4. The cycloalkylammonium 2-(1-hydroxypentyl) benzoate salt or isomers thereof according to claim 1, **characterized in that** said cycloalkylammonium 2-(1-hydroxypentyl) benzoate salt comprises the following compounds:

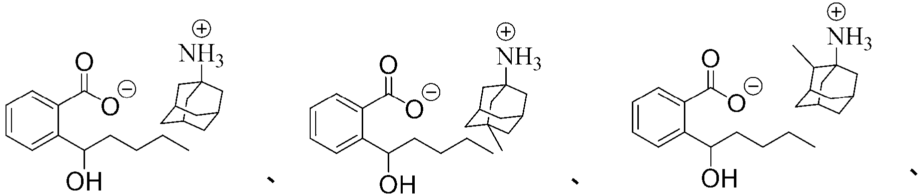

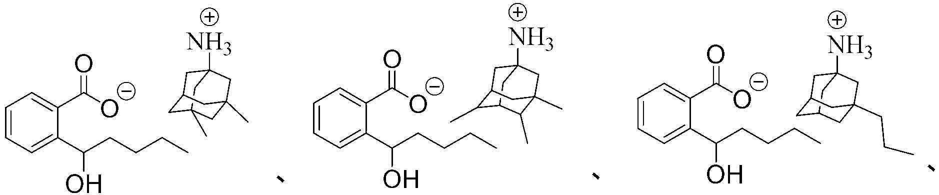

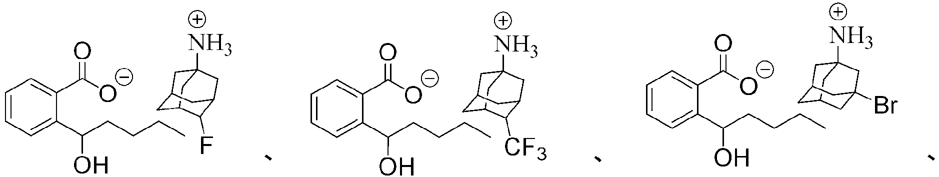

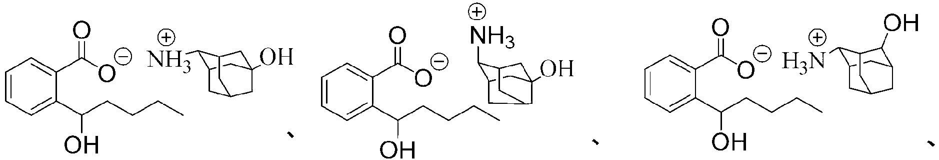

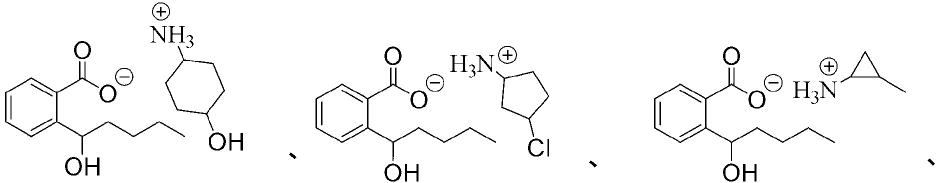

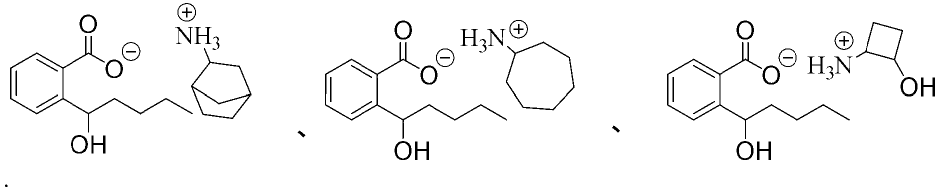

5. The cycloalkylammonium 2-(1-hydroxypentyl) benzoate salt or isomers thereof according to any one of claims 1 to 4, **characterized in that** the hydrogen atoms in the compound may be substituted with one or more deuterium atoms.

6. A method for preparing the cycloalkylammonium 2-(1-hydroxypentyl) benzoate salt or isomers thereof according to any one of claims 1 to 5, **characterized by** comprising the following step: 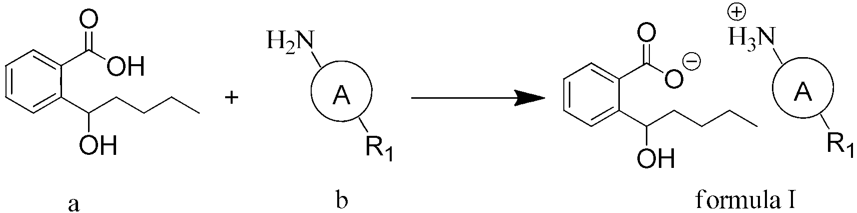
allowing compound a and compound b to undergo a salt formation reaction in the presence of an organic solvent, to yield a compound of Formula I;
wherein the cycle A and R₁ are defined as in claim 1.

7. The method according to claim 6, **characterized in that** said organic solvent comprises, but is not limited to, an ether- or ester-based solvent; preferably, said ether-based solvent comprises, but is not limited to, ethyl ether or methyl tert-butyl ether; said ester-based solvent comprises, but is not limited to, ethyl acetate, isopropyl acetate, or butyl acetate; and/or the salt is formed at a temperature of 0 to 50°C, preferably 10 to 30°C.

8. A pharmaceutical composition comprising the cycloalkylammonium 2-(1-hydroxypentyl) benzoate salt or isomers thereof according to any one of claims 1 to 5, **characterized in that** said pharmaceutical composition further comprises a pharmaceutically acceptable carrier.

9. Use of the cycloalkylammonium 2-(1-hydroxypentyl) benzoate salt or isomers thereof according to any one of claims 1 to 5 in the preparation of a medicament for the treatment and/or prevention of ischemic cardiovascular or cerebrovascular diseases.

10. The use according to claim 9, **characterized in that** said ischemic cardiovascular or cerebrovascular diseases include, but are not limited to, mild, moderate, or severe acute ischemic stroke, cerebral thrombosis, cerebral embolism, coronary artery disease, angina pectoris, or myocardial infarction; preferably, moderate or severe acute ischemic stroke; and more preferably improvement of neurological deficits in patients with acute ischemic stroke.
